# EUROPEAN PATENT SPECIFICATION

(11) **EP 0 709 814 B1**
(45) Date of publication and mention of the grant of the patent: **04.04.2001**
(21) Application number: 95202830.6
(22) Date of filing: 19.10.1995
(51) Int. Cl.: G08C 17/00, G01N 27/12

(54) **Method and device for determining the moisture-content of construction material**
Verfahren und Vorrichtung zum Bestimmen des Feuchtigkeitsgehaltes von Baumaterial
Procédé et dispositif pour déterminer le degré d'humidité des matériaux de construction

(30) Priority: 24.10.1994 NL 9401764
(43) Date of publication of application: 01.05.1996
(73) Proprietor: Isoplan B.V., 7301 BJ Apeldoorn (NL)
(72) Inventor: Cornelissen, Johan Allerd, NL-7322 GS Apeldoorn (NL)
(74) Representative: Van Breda, Jacobus

(56) References cited:
- EP-A- 0 601 739
- DE-A- 4 440 680
- GB-A- 2 245 976

## Description

The present invention relates to a method for the determination of the moisture content of construction material, in particular that of wooden casings, doors and the like, wherein a measuring instrument provided with measuring organs is brought into contact with the construction material, whereby the measured value of said measuring instrument can be read out by means of a separate read only unit giving an indication of the moisture content of the construction material. The invention also relates to a measuring device for the application of the method.

At present it is usual that, for instance in the case of wooden casings, a surveyor periodically carries out manual determination and inspection of the moisture content of the wood. The measuring instrument is then placed, in a somewhat arbitrary manner, into a number of successive casing joints. Because the measuring instrument is introduced manually, the measurement yields an unreliable result, as such measuring is only rarely carried out in the same way as an earlier one. Also, the way the measuring instrument is applied may vary from person to person. Such measurements are therefore to a large extent inaccurate. In addition, the measuring is lengthy and time consuming and in most cases the wood or the coat of paint is damaged.

GB-A-2 245 976 discloses a moisture sensor having two conductors which are applied on an absorbent material such as wood. The moisture is measured by determining the electrical resistance between the conductors. The sensor is provided with a sleeve leaving an end face of the absorbent material exposed which allows for the ingress and egress of moisture. The known sensor element is suggested to be located permanently or semi permanently in position in a piece of wood, the moisture content of which is to be monitored. A cable connects the conductors of the sensor with a moisture meter; hence the construction material in which the known sensor is applied is visibly affected by the application of the known sensor.

The object of the present invention is to provide a method and device of the type mentioned in the preamble, whereby the determination of the moisture content can take place quickly and reliably, without damaging the surface of the construction material.

To this end the method according to the invention is characterized in that the measuring instrument is applied completely and permanently under the surface of the construction material with wireless transmission of the measured values to the read only unit, and that the measuring organs are shapen in the form of at least two conductive organs placed at a distance from each other to measure the conduction of the construction material between these conductive organs. The measured value of said measuring instrument can be read out by means of a separate read only unit.

The advantage of the permanent presence of a measuring instrument in the construction material in combination with a separate read only unit is, that the periodic determination of the moisture content is reliable: measurement takes place every time in the same manner and is independent of the performing person. Moreover, the measuring instrument need no longer be introduced into the construction material with every measurement. This will therefore save time and costs.

It is preferable to activate the measuring instrument via the read only unit before measuring is initiated. This may be realized, by activating the measuring instrument via the read only unit by means of external radiation from an electromagnetic field. Immediately after activation measurement takes place and also almost immediately the measured values can be read off the read only unit, so that no other component is necessary for switching on or activating the measuring instrument.

It is advantageous if the measuring instrument transmits its identification code to the read only unit. In this way every measuring instrument hidden in the construction material is identifiable from the outside. If, in addition, the identification code is coupled to the construction material sort, the moisture content is automatically calculated on the basis of the measurement, without first having to enter the construction material sort.

It is also advantageous if the temperature is measured and preferably if the temperature is measured by the measuring instrument and passed through to the read only unit. It is hereby preferable if on the read only unit the measured moisture content is corrected for the measured temperature. This makes the measurement more accurate, and the final value of the moisture content corrected for the temperature, in the case of wood the so-called standardized wood moisture value, can be directly read off the read only unit.

The measured values are preferably stored in the read only unit. In this way the measured values can be collected and presented, for instance in an account per casing/apartment and/or building complex. This also makes it possible to obtain a clear and periodic account of the moisture content of the material. Possible damage can be spotted early on, so that further damage and rot can be prevented. This means a decrease in maintenance costs and a longer life span of the construction material.

It is also possible to have the read only unit pass through the stored measured values to a computer. In this way the measured values can be read in and analyzed at any desired time and on a location other than the place of measurement. The measured values may, for instance, undergo all kinds of statistic operations.

When used with wood, the measuring instrument is preferably applied in untreated wood. This avoids the conductivity and consequently the moisture content to be influenced by salts that might be present in the impregnating agent.

The measuring device already mentioned in the preamble comprises a measuring instrument provided with measuring organs that must come in contact with the construction material in order to provide measured values representing the moisture content in the construction material, and which comprises a separate read only unit for reading out the measured values from the measuring instrument. According to the invention the measuring device is applied completely and permanently under the surface of the construction material, whereby the transmitted values are transmitted wirelessly to the read only unit, and the measuring organs of the measuring instrument have the form of at least two conductive organs placed at a distance from each other, measuring the conductivity of the construction material between these two conductive organs. Hereby it is advantageous if the measuring instrument is accommodated almost entirely, with the exception of at least part of the measuring organs, in a tightly sealed glass house. This makes the measuring instrument resistant to corrosion from the outside and lengthens its life span.

The invention will be elucidated below referring to the accompanying drawings, which show schematically an embodiment of the invention.

Fig. 1 is a front view of a mounted wooden window casing into which the measuring instrument is fitted.

Fig. 2 illustrates the measuring device according to the invention, whereby the window casing is shown in cross-section according to the line II-II in Fig. 1, and the read only unit is indicated in the form of a block diagram.

Fig. 3 is a block diagram of the measuring instrument applied in the method according to the invention.

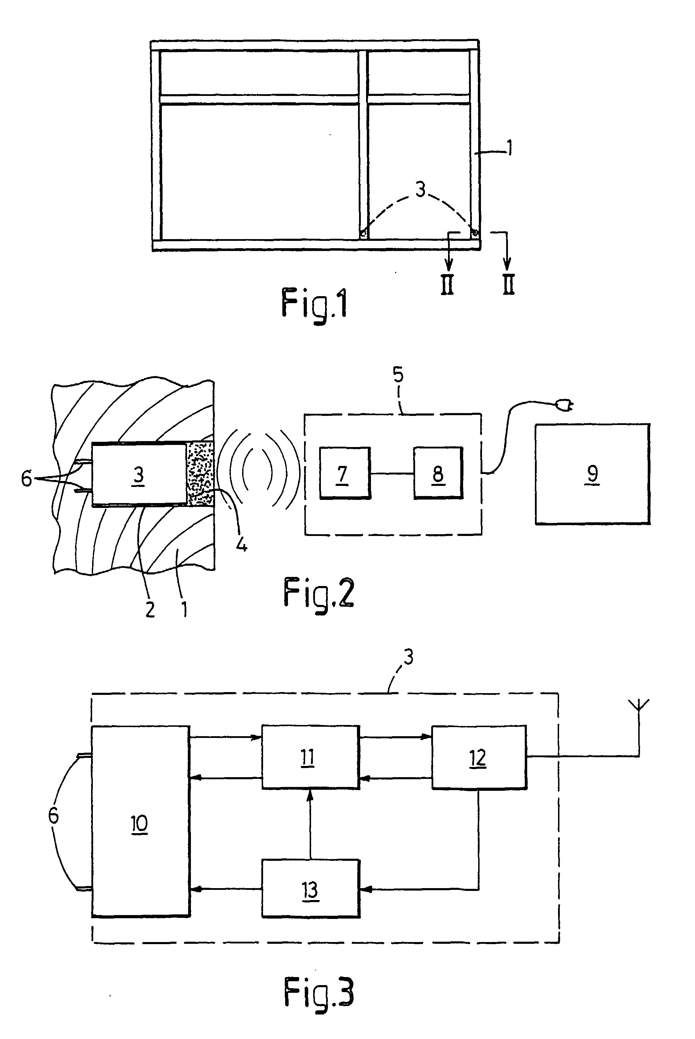

As is shown in Figs. 1 and 2 of the illustrated embodiment, a hole 2 has been made into a casing 1. In the hole 2 a measuring instrument 3 is mounted and the hole 2 is sealed to the outside by means of a special compound 4. As is shown in Fig. 1, the measuring instrument is fitted in the places most susceptible to wood rot, that is to say near joints in the casing 1. Making the hole 2 and subsequently fitting the measuring instrument 3 in the hole 2 and finally sealing the hole 2 by means of the compound 4, occurs, in the case of new casings, at the joinery works before the parts of the casing 1 are fitted to each other. These operations may, of course, also be carried out during repair or maintenance work on existing casings, so that existing casings and the like may be provided with a measuring instrument later on. Fig. 2 shows that the hole 2 is made from the outside of the casing 1 such that it runs more or less perpendicular in relation to the outside. The measuring device 3 is placed into the hole 2 such that two metal pins 6 at the top side of the measuring instrument 3, serving as measuring and conducting organs, protrude into the casing 1.

Fig. 2 further shows, that during the determination of the wood's moisture content a read only unit 5 is kept at a distance to the measuring instrument 3 concealed in the casing 1. This distance may be, for instance 5-10 cm. The exact position of the measuring instrument 3 in the casing 1 is, of course, known to the read only unit 5 or to the person measuring and operating the read only unit 5. The read only unit 5 is provided with an RF-unit 7 and a digital measuring system 8. In the RF-unit 7 an electromagnetic field is generated supplying the measuring instrument 3 with the necessary energy for carrying out a measurement and sending the measured values back to the RF-unit 7 in the read only unit 5. The measurement consists of measuring, for instance with the aid of direct current, the electric conductivity of the wood between the pins 6 of the measuring instrument 3. Further, the ambient temperature is measured by means of the read only unit 5, but preferably, the temperature of the wood is measured by means of the measuring instrument 3. These measured data plus an identification code of the measuring instrument 3 are returned in the form of a signal to the RF-unit 7 in the read only unit 5. This signal is passed through digitally to the digital measuring system 8. Here the received signal is analyzed and converted into the wood's moisture content. When the identification code of the measuring instrument 3 is coupled to the kind of wood of the casing 1, the measured temperature allows automatic computing of a wood moisture content for a specific kind of wood at a certain temperature. This is the standardized wood moisture value. A visual display unit at the outside of the read only unit 5 shows the wood moisture value and the identification code of the measuring instrument 3. At the outside of the read only unit 5 there is also (not shown) an input control panel for entering adjustment data and for starting the measurements. The read only unit 5 is also provided with an internal memory (not shown) for storing the measured values and can be connected to a computer 9 for the presentation of the stored measured values. This computer 9 may of course be placed at a considerable distance from the casing 1 and the read only unit 5. With the aid of this computer 9 the measured values can be summarized while, at any desired moment, all kinds of statistic operations may be carried out on these measured values.

With reference to Fig. 3 the workings of the measuring instrument 3 will now be further elucidated. The measuring instrument 3 is enclosed in a glass housing (not shown) which is sealed by melting. Only the pins 6 protrude from the glass housing. The measuring instrument 3 is provided with a moisture measuring unit 10 comprised of an electronic circuit measuring the moisture content of the wood and converting it into an electric signal. This signal is converted, in a digital IC 11, into a digital bit stream. This bit stream is passed to a transmission output stage 12, comprised of a transistor which, for instance on a frequency of 30MHz, sends the signals to the read only unit 5. The measuring instrument 3 is also provided with a power supply 13 comprised of an induction coil, a diode and a condenser. The total measuring instrument 3 is more or less a cylindrical cartridge having a diameter of between 6-10 mm and a maximum length of about 50 mm. Measurements are carried out at a minimum distance of 5 mm from the surface and in the case of casings preferably at a distance of about 50 mm into the wood.

From the foregoing it is clear that the invention provides a method and accessory measuring instrument for the determination of the moisture content of wood, which is fast and reliable and whereby the wood surface is not damaged. In addition, the moisture in the wood of, for instance a casing can periodically be summarized, so that possible damage may be spotted early and the casing be repaired in time.

The invention is not limited to the embodiments shown in the drawing and described in the foregoing, which embodiments may, within the scope of the invention, be varied in different ways. Using the method according to the invention it is also possible to determine the moisture content of the wood by measuring the dielectric constant of the wood. It is also possible to measure the moisture content of the air present in the wood as indication of the moisture content of the wood. The measuring instrument may then, for instance, be placed in a cavity inside the wood. In addition to measurements in wood casings and doors, similar measurements according to the invention can also be carried out in wooden construction parts such as posts and beams or in constructions made entirely of wood, or even in parts made of other construction material such as stone and concrete. In this latter example, for instance, rising damp may be spotted, and generally the behaviour in time of construction materials can be monitored.

## Claims

1. A method for the determination of the moisture content of construction material (1), in particular that of wooden casings, doors and the like, wherein a measuring instrument (3) provided with measuring organs (6) is brought into contact with the construction material (1), whereby the measured values of said measuring instrument can be read out by means of a separate read only unit (5) giving an indication of the moisture content of the construction material (1), **characterized** in that the measuring instrument (3) is applied completely and permanently under the surface of the construction material (1), that the measured values are transmitted wirelessly (4) to the read only unit (5), and that the measuring organs (6) are shapen in the form of at least two conductive organs placed at a distance from each other to measure the conduction of the construction material between these conductive organs (6).

2. A method according to claim 1, **characterized** in that the measuring instrument (3) in the construction material (1) takes measurements at a minimum distance of 5 mm from the surface.

3. A method according to any one of the preceding claims, **characterized** in that the measuring instrument (3) is activated via the read only unit (5) before measuring is initiated.

4. A method according to any one of the preceding claims, **characterized** in that the measuring instrument (3) passes its identification code to the read only unit (5).

5. A method according to claim 7, **characterized** in that the identification code is linked to the construction material sort (1).

6. A method according to any one of the preceding claims, **characterized** in that the temperature is measured by the measuring instrument (3) and passed through to the read only unit (5), and that the moisture content measured on the read only unit (5) is corrected for the measured temperature.

7. A method according to any one of the preceding claims, **characterized** in that the measured values are stored in the read only unit (5).

8. A method according to any one of the preceding claims, **characterized** in that the measured values are passed through by the read only unit (5) to a computer (9).

9. A method according to any one of the preceding claims for application with wood, **characterized** in that the measuring instrument (3) is applied in untreated wood (1).

10. A measuring device for the determination of the moisture content of construction material (1) comprising a measuring instrument (3) provided with measuring organs (6) which are brought into contact with the material (1) and a separate read only unit (5) for reading out the measured values from the measuring instrument (3), **characterized** in that,
- the measuring device (3) is applied completely and permanently under the surface of the construction material (1) and
- the measured values are transmitted wirelessly to the read only unit (5) and
- the measuring organs (6) have the form of at least two conductive organs (6) placed at a distance from each other to measure the conductivity of the construction material (1) between these conductive organs (6).

11. A measuring device according to claim 10, **characterized** in that the conductive organs (6) have the form of metal pins.

12. A measuring device according to any one of claims 10-11, **characterized** in that the read only unit (5) is provided with an RF-unit (7) for activating and reading out the measuring instrument (3), a digital measuring system (8) to convert the received signals into the moisture content, with a display and an interface for the coupling to a computer (9).

13. A measuring device according to any one of claims 10-12, **characterized** in that the measuring instrument (3) is almost entirely, with the exception of at least part of the measuring organs, accommodated in a tightly sealed glass house.

14. A measuring device according to any one of claims 10-13, **characterized** in that the measuring instrument (3) is in essence cylindrical in shape.

15. A measuring device according to claim 14, **characterized** in that the diameter of the measuring instrument (3) is between 6-10 mm and the maximum length is about 50 mm, whereby the measuring organs (6) are attached to the top side of the measuring instrument (3).

## Patentansprüche

1. Verfahren zur Bestimmung des Feuchtigkeitsgehaltes von Baumaterial (1), insbesondere desjenigen von Holzverschalungen, Türen und dergleichen, wobei ein Meßinstrument (3), das mit Meßorganen (6) ausgestattet ist, mit dem Baumaterial (1) in Kontakt gebracht wird, wobei die gemessenen Werte des Meßinstrumentes mittels einer separaten Nurleseeinheit (5), die ein Zeichen für den Feuchtigkeitsgehalt des Baumaterials (1) angibt, ausgelesen werden, **dadurch gekennzeichnet,** daß das Meßinstrument (3) vollständig und dauerhaft unter der Oberfläche des Baumaterials (1) angebracht wird, daß die gemessenen Werte drahtlos (4) zu der Nurleseeinheit (5) übermittelt werden und daß die Meßorgane (6) in Form von mindestens zwei leitenden Organen geformt sind, die zur Messung der Leitfähigkeit des Baumaterials zwischen diesen leitenden Organen (6) beabstandet voneinander angeordnet sind.

2. Verfahren nach Anspruch 1, **dadurch gekennzeichnet,** daß das Meßinstrument (3) in dem Baumaterial (1) Messungen bei einem minimalen Abstand von 5 mm von der Oberfläche vornimmt.

3. Verfahren nach einem der vorhergehenden Ansprüche, **dadurch gekennzeichnet,** daß das Meßinstrument (3) vor Beginn der Messung über die Nurleseeinheit (5) aktiviert wird.

4. Verfahren nach einem der vorhergehenden Ansprüche, **dadurch gekennzeichnet,** daß das Meßinstrument (3) seinen Erkennungscode an die Nurleseeinheit (5) übermittelt.

5. Verfahren nach Anspruch 7, **dadurch gekennzeichnet,** daß der Erkennungscode mit der Baumaterialsorte zusammenhängt.

6. Verfahren nach einem der vorhergehenden Ansprüche, **dadurch gekennzeichnet,** daß die Temperatur durch das Meßinstrument (3) gemessen und an die Nurleseeinheit (5) weitergeleitet wird und daß der auf der Nurleseeinheit (5) gemessene Feuchtigkeitsgehalt für die gemessene Temperatur korrigiert wird.

7. Verfahren nach einem der vorhergehenden Ansprüche, **dadurch gekennzeichnet,** daß die gemessenen Werte in der Nurleseeinheit (5) gespeichert werden.

8. Verfahren nach einem der vorhergehenden Ansprüche, **dadurch gekennzeichnet,** daß die gemessenen Werte über die Nurleseeinheit (5) an einen Computer (9) weitergeleitet werden.

9. Verfahren nach einem der vorhergehenden Ansprüche zur Anwendung mit Holz, **dadurch gekennzeichnet,** daß das Meßinstrument (3) in unbehandeltem Holz (1) angewandt wird.

10. Meßvorrichtung zur Bestimmung des Feuchtigkeitsgehaltes von Baumaterial (1), umfassend ein Meßinstrument (3), das ausgestattet ist mit Meßorganen (6), die mit dem Material (1) in Kontakt gebracht werden, und mit einer separaten Nurleseeinheit (5) zum Auslesen der gemessenen Werte aus dem Meßinstrument (3), **dadurch gekennzeichnet,** daß
- die Meßvorrichtung (3) vollständig und dauerhaft unter der Oberfläche des Baumaterials (1) angewandt wird und
- die gemessenen Werte drahtlos an die Nurleseeinheit (5) übermittelt werden und
- die Meßorgane (6) die Form von mindestens zwei leitenden Organen (6) einnehmen, die zur Messung der Leitfähigkeit des Baumaterials zwischen diesen leitenden Organen (6) beabstandet voneinander angeordnet sind.

11. Meßvorrichtung nach Anspruch 10, **dadurch gekennzeichnet,** daß die leitenden Organe (6) die Form von Metallstiften einnehmen.

12. Meßvorrichtung nach einem der Ansprüche 10-11, **dadurch gekennzeichnet,** daß die Nurleseeinheit (5) mit einer RF-Einheit (7) zum Aktivieren und Auslesen des Meßinstrumentes (3), einem digitalen Meßsystem (8) zur Umwandlung des empfangenen Signals in den Feuchtigkeitsgehalt, mit einer Anzeige und einer Schnittstelle zur Kopplung mit einem Computer ausgestattet ist.

13. Meßvorrichtung nach einem der Ansprüche 10-12, **dadurch gekennzeichnet,** daß das Meßinstrument (3) fast vollständig, mit Ausnahme von mindestens einem Teil der Meßorgane, in einem fest verschlossenen Glasgehäuse eingeschlossen ist.

14. Meßvorrichtung nach einem der Ansprüche 10-13, **dadurch gekennzeichnet,** daß das Meßinstrument (3) praktisch zylindrisch geformt ist.

15. Meßvorrichtung nach Anspruch 14, **dadurch gekennzeichnet,** daß der Durchmesser des Meßinstrumentes (3) zwischen 6-10 mm und die maximale Länge etwa 50 mm betragen, wobei die Meßorgane (6) auf der Oberseite des Meßinstrumentes (3) befestigt sind.

## Revendications

1. Procédé pour déterminer la teneur en humidité d'un matériau de construction (1), en particulier celui de caisses en bois, de portes en bois et analogues, selon lequel un instrument de mesure (3) pourvu d'organes de mesure (6) est placé en contact avec le matériau de construction (1), ce qui permet la lecture des valeurs mesurées dudit instrument de mesure au moyen d'une unité séparée de lecture seule (5), fournissant une indication de la teneur en humidité du matériau de construction (1),
caractérisé en ce que l'instrument de mesure (3) est appliquée d'une manière complète et permanente au-dessous de la surface du matériau de construction (1), que les valeurs mesurées sont transmises sans câble (4) à l'unité à lecture seule (5) et que les organes de mesure (6) sont agencés sous la forme d'au moins deux organes conducteurs situés à distance l'un de l'autre pour mesurer la conduction du matériau de construction entre ces organes conducteurs (6).

2. Procédé selon la revendication 1, caractérisé en ce que l'instrument de mesure (3) placé dans le matériau de construction (1) exécute des mesures à une distance minimale de 5 mm à partir de la surface.

3. Procédé selon l'une quelconque des revendications précédentes, caractérisé en ce que l'instrument de mesure (3) est activé au moyen de l'unité à lecture seule (5) avant le déclenchement de la mesure.

4. Procédé selon l'une quelconque des revendications précédentes, caractérisé en ce que l'instrument de mesure (3) transmet son code d'identification à l'unité à lecture seule (5).

5. Procédé selon la revendication 7, caractérisé en ce que le code d'identification est associé au type de matériau de construction (1).

6. Procédé selon l'une quelconque des revendications précédentes, caractérisé en ce que la température est mesurée par l'instrument de mesure (3) et est transmise à l'unité de lecture seule (5) et que la teneur en humidité mesurée dans l'unité de lecture seule (5) est corrigée pour tenir compte de la température mesurée.

7. Procédé selon l'une quelconque des revendications précédentes, caractérisé en ce que les valeurs mesurées sont mémorisées dans l'unité de lecture seule (5).

8. Procédé selon l'une quelconque des revendications précédentes, caractérisé en ce que les valeurs mesures sont transmises par l'unité de lecture seule (5) à un ordinateur (9).

9. Procédé selon l'une quelconque des revendications précédentes destiné à être utilisé avec du bois, caractérisé en ce que l'instrument de mesure (3) est appliqué dans du bois non traité (1).

10. Dispositif de mesure pour déterminer la teneur en humidité d'un matériau de construction, comprenant un instrument de mesure (3) pourvu d'organes de mesure (6), qui sont placés en contact avec le matériau (1), et une unité séparée de lecture seule (5) pour lire les valeurs mesurées fournies par l'instrument (3), caractérisé en ce que
- le dispositif de mesure (3) est appliqué d'une manière complète et permanente au-dessous de la surface du matériau de construction (1), et
- les valeurs mesurées sont transmises sans câble (4) à une unité de lecture seule (5) et
- les organes de mesure (6) possèdent la forme d'au moins deux organes conducteurs situés à distance l'un de l'autre pour mesurer la conduction du matériau de construction (1) entre ces organes conducteurs (6).

11. Dispositif de mesure selon la revendication 10, caractérisé en ce que les organes conducteurs (6) possèdent la forme de broches métalliques.

12. Dispositif de mesure selon l'une quelconque des revendications 10 et 11, caractérisé en ce que l'unité de lecture seule (5) est pourvue d'une unité HF (7) servant à activer et lire l'instrument de mesure (3), d'un système de mesure numérique (8) pour convertir les signaux reçus en la teneur en humidité, d'un dispositif d'affichage et d'une interface pour le couplage à un ordinateur (9).

13. Dispositif de mesure selon l'une quelconque des revendications 10-12, caractérisé en ce que l'instrument de mesure (3) est presque logé entièrement, à l'exception d'au moins une partie des organes de mesure, dans un logement en verre scellé d'une manière étanche.

14. Dispositif de mesure selon l'une quelconque des revendications 10-13, caractérisé en ce que l'instrument de mesure (3) possède une forme essentiellement cylindrique.

15. Dispositif de mesure selon la revendication 14, caractérisé en ce que le diamètre de l'instrument de mesure (3) est compris entre 6 et 10 mm et que la longueur maximale est égale au maximum à 50 mm, les organes de mesure (6) étant fixés sur la face supérieure de l'instrument de mesure (3).
